# EUROPEAN PATENT SPECIFICATION

(11) **EP 0 583 794 B1**
(45) Date of publication and mention of the grant of the patent: **30.11.2005**
(21) Application number: 93113917.4
(22) Date of filing: 23.09.1987
(51) Int. Cl.: C12N 15/00, A61K 39/104, A61K 38/00

(54) **Recombinant pseudomonas exotoxin: construction of an active immunotoxin with low side effects**
Rekombinantes Exotoxin von Pseudomonas: Konstruktion eines aktiven Immunotoxins mit schwachen Nebenwirkungen
Exotoxine récombinante de Pseudomonas: construction d'une immunotoxine active avec effets secondaires faibles

(30) Priority: 24.09.1986 US 911227
(43) Date of publication of application: 23.02.1994
(62) Divisional of application: 87113929.1
(73) Proprietor: Pastan, Ira, Potamac Maryland 20854 (US); Fitzgerald, David J., Silver Spring Maryland 20902 (US); Adhya, Sankar, Gaithersbourg Maryland 20878 (US)
(72) Inventor: Pastan, Ira, Potamac Maryland 20854 (US); Fitzgerald, David J., Silver Spring Maryland 20902 (US); Adhya, Sankar, Gaithersbourg Maryland 20878 (US)
(74) Representative: Grünecker, Kinkeldey, Stockmair & Schwanhäusser Anwaltssozietät

(56) References cited:
- WO-A-86/00090
- PROC. NATL. ACAD. SCI. USA vol. 81 , 1984 pages 2645 - 2649 G. GRAY ET AL. 'Cloning, nucleotide sequence and ecpression in E. coli of the exotoxin A structural gene of P. aeruginosa'
- CANCER RESEARCH vol. 45 , 1985 pages 751 - 757 R. PIRKER ET AL. 'Anti-transferrin receptor antibody linked to Pseudomonas exotoxin as a model immunotoxin'
- INFECT. IMMUN. vol. 52, no. 3 , 1986 pages 756 - 762 J. CHIA ET AL. 'Functionally distinct monoclonal antibodies reactive with enzymatically active and binding domains of P. aeruginosa toxin A'
- J. BACTERIOL. vol. 159, no. 2 , 1984 pages 683 - 687 M. MOZOLA ET AL. 'Cloning and expression of a gene segment encoding the enzymatic moeity of P. aeruginosa exotoxin A'
- INFECT. IMMUN. vol. 28, no. 2 , 1980 pages 494 - 501 S. LORY ET AL. 'Expression of enzymatic activity by exotoxin A of p. aeruginosa'

### SEQUENCE LISTING

(1) GENERAL INFORMATION:
   (i) APPLICANT:
      (A) NAME: Pastan, Ira
      (B) STREET: 11710 Beall Mountain Road
      (C) CITY: Potamac
      (D) STATE: Maryland
      (E) COUNTRY: USA
      (F) POSTAL CODE (ZIP): 20854

      (A) NAME: Fitzgerald, David J.
      (B) STREET: 1731 Ladd Street
      (C) CITY: Silver Spring
      (D) STATE: Maryland
      (E) COUNTRY: USA
      (F) POSTAL CODE (ZIP): 20902

      (A) NAME: Adhya, Sankar
      (B) STREET: 1440 Kings Grant Street
      (C) CITY: Gaithersburg
      (D) STATE: Maryland
      (E) COUNTRY: USA
      (F) POSTAL CODE (ZIP): 20878
   (ii) TITLE OF INVENTION: Recombinant Pseudomonas Exotoxin: construction of an active immunotoxin with low side effects
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25 (EPO)
(2) INFORMATION FOR SEQ ID NO: 1:
   (i) SEQUENCE CHARACTERISTICS:
      (A) LENGTH: 9 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: YES
   (iii) ANTI-SENSE: NO
   (xi) SEQUENCE DESCRIPTION: SEQ ID NO: 1:
   TATGAAAAA 9
(2) INFORMATION FOR SEQ ID NO: 2:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: YES
   (iii) ANTI-SENSE: NO
   (xi) SEQUENCE DESCRIPTION: SEQ ID NO: 2:
   GATCTTTTTC A 11
(2) INFORMATION FOR SEQ ID NO: 3:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: YES
   (iii) ANTI-SENSE: NO
   (xi) SEQUENCE DESCRIPTION: SEQ ID NO: 3:
   TATGGCCGAA GAAGCTTT 18
(2) INFORMATION FOR SEQ ID NO: 4:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: YES
   (iii) ANTI-SENSE: NO
   (xi) SEQUENCE DESCRIPTION: SEQ ID NO: 4:

## Description

### Background of the Invention

Toxins are extremely potent cell-killing agents that are responsible for many human diseases. Because of their high activity, these agents have been attached to monoclonal antibodies in order to form cytotoxic agents (immunotoxins) which specifically bind to target cells. These immunotoxins are, therefore, most useful in cancer therapy.

Pseudomonas exotoxin A (PE) is an extremely active monomeric protein (molecular weight 66Kd), secreted by Pseudomonas aeruginosa, which inhibits protein synthesis in eukaryotic cells through the inactivation of elongation factor 2 (EF-2) by catalyzing its ADP-ribosylation (catalyzing the transfer of the ADP ribosyl moiety of oxidized NAD onto EF-2).

The intoxication process is believed to proceed by the following steps: First, PE binds to cells through a specific receptor on the cell surface. Next, the PE-receptor complex is internalized into the cell. Finally, PE is transferred to the cytosol where it enzymatically inhibits protein synthesis. The transfer process is believed to occur from an acidic compartment, since cellular intoxication is prevented by weak bases such as NH₄⁺, which raises the pH in acidic vesicles. Upon exposure to acidic conditions, the hydrophobic domain of PE enters into the membrane, resulting in the formation of a channel through which the enzymatic domain, in extended form, passes into the cytosol.

Gray et al., PNAS 81 (1984), 2645 to 2649 relates to the cloning, nucleotide sequence and expression in *E. coli* of the exotoxin A structural gene of *P*. *aeruginosa.* D1 describes the recombinant expression in *E. coli* of beta-galactosidase fusion proteins comprising amino acids residues 308 to 613 and 492 to 613, respectively, of *P. aeruginosa* exotoxin A. Both fusion proteins have been found to exhibit ADP ribosylation activity.

U.S. patent 4,545,985 teaches that pseudomonas toxin can be chemically coupled to an antibody or growth factor. However, these chemically linked toxins have been shown to have undesirable levels of toxicity. It would be useful, therefore, to have toxins highly targeted for specific cell types without the generalized cell killing normally associated with these toxins.

PE-containing immunotoxins are constructed by first reacting native PE with iminothiolane. This reaction serves both to introduce two new sulfhydryl groups used for coupling an antibody to the toxin, and to inactivate the binding of PE to its own receptor. This approach relies on the chemical inactivation of PE-binding sites in order to minimize undesirable side effects due to the binding of PE to cells with PE receptors. While this approach has been reasonably successful in producing a specific cell killing reagent in tissue culture and in tumor-bearing mice, it has not been possible to administer more than 2 ug of PE immunotoxin to a 20 gram mouse or 1 mg to a 3 kilogram monkey or 4 mg to an adult human, due to the toxic side effects of the immunotoxin. It is therefore desirable to be able to administer larger amounts of immunotoxins to achieve greater killing of tumor cells. The present invention fulfills this desire by providing an immunotoxin with high potency and low toxicity. Furthermore, to overcome the above-noted reliance on chemical inactivation of the PE binding sites, the present invention incorporates recombinant DNA techniques to clone the complete toxin gene (or segments of it) in order to express at high levels the full length toxin molecule (or portions of it) containing different functional domains, including one which lacks the cell binding domain. See example 1 for a comparative examination of these clones.

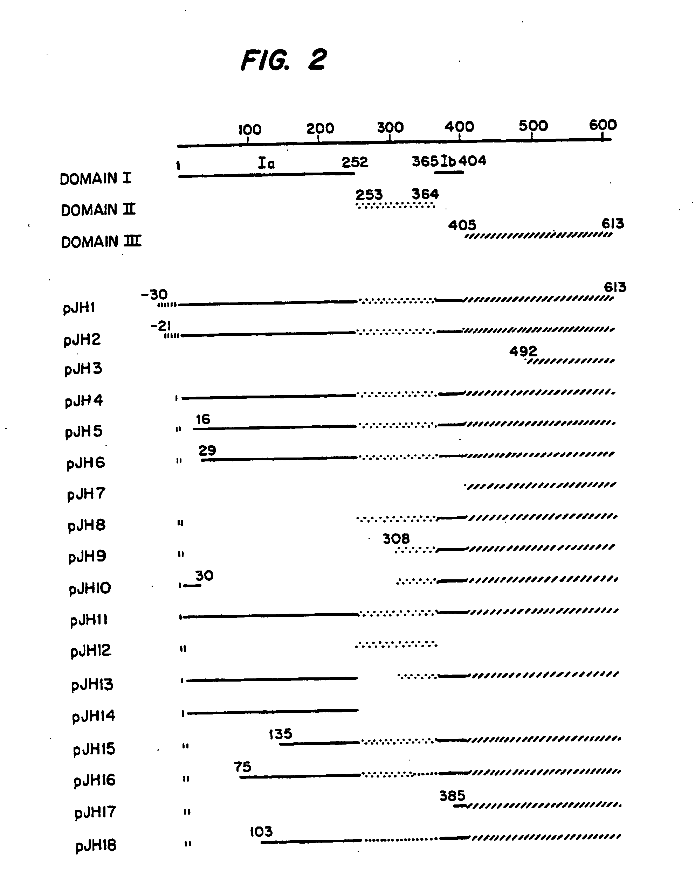

The three-dimensional structure of PE has been determined by x-ray crystallography. As shown in Figure 2, the PE molecule contains three structurally distinct domains: Domain I contains amino acid residues 1 to 252 (Domain Ia), and 365 to 404 (Domain Ib); domain II contains amino acid residues 253 to 364; and domain III contains amino acid residues 405 to 613.

Plasmids have been constructed which express various portions of the PE molecule, providing the ability to correlate different structural domains with various functional activities and to determine (1) which portions of the molecule are responsible for cell recognition (binding), (structural domain I, amino acid 1-252); (2) which portion is required for enzymatic activity (ADP ribosylating activity, domain III plus a portion of domain Ib) (amino acid 385-613); (3) which portion is responsible for translocation across cell membrane (domain II). The evidence that structural domain Ia is involved in cell recognition is that proteins produced by plasmids without domain Ia but containing domain II, Ib and III, are not cytotoxic by themselves and do not show competitive inhibition of the cytotoxicity of intact toxins, whereas a plasmid encoding domain Ia, but missing other domains, blocked PE cytotoxicity of sensitive cells. PE from plasmids with a deletion of the first half of structural domain II exhibit both PE blocking activity and ADP-ribosylation activity, but these molecules lost all cell killing activity. Plasmids which encode only structural domain III produce large amounts of the protein, but lack detectable enzymatic activity (ADP-ribosylation). However, plasmids which encode all of structural domain III plus adjacent amino acids from structural domain Ib express large amounts of the protein and their ADP-ribosylation activity is high.

Based on the three dimensional structure of PE, plasmids have been constructed which express different portions of PE. The protein pattern of the cells expressing the different constructions was analyzed by SDS gel electrophoresis and the ADP ribosylating activity of the recombinant toxins was measured. Of these plasmids (described in Example 1) plasmid pJH8, containing amino acids 253 to 613 (Domains Ib, II, and III), and plasmid pJH17, containing amino acids 385 to 613 (Domain III, plus 20 adjacent amino acids from Domain Ib), are able to encode large amounts of modified PE exhibiting low toxicity to human cells, but remaining enzymatically very active.

Taken together, the plasma patterns indicate that structural domain Ia is a receptor binding domain, that the first half of structural domain II is required for translocation of the toxin from a host cell's endocytic vesicle to the cytosol, and that structural domain III alone is not sufficient to express full ADP-ribosylation activity.

When administered to animals, PE characteristically produces death due to liver failure. Immunotoxins made with PE also attack the liver and, when given in large amounts, produce death due to liver toxicity. The experiments shown in Table III indicate that Domain Ia is responsible for cell binding and indicate that a PE molecule in which Domain Ia is deleted is less toxic to mice than native PE. The data shown in Example 4 support this conclusion, indicating that modified PE is at least 200-fold less toxic to mice than native PE. Example 5 shows that when the protein made by pJH6 is purified and coupled to an antibody to the human transferrin receptor, an immunotoxin is created that is approximately as active as an immunotoxin containing native PE but is 100-fold less toxic on nontarget cells (mouse cells).

One aspect of the present invention, therefore, is that PE molecules with a deletion of Domain Ia are effective immunotoxins with diminished side effects including diminished liver toxicity.

According to one aspect the present invention provides a targeting carrier-toxin conjugate suitable for use with humans or mammals comprising a toxin fused to a targeting carrier, wherein said toxin is a modified Pseudomonas exotoxin selected from:
(i) an exotoxin comprising the amino acid residues 1 to 30 and 308 to 613 of Pseudomonas exotoxin;
(ii) an exotoxin comprising the amino acid residues 1 to 252 and 308 to 613 of Pseudomonas exotoxin; and
(iii) an exotoxin comprising the amino acid residues 385 to 613 of Pseudomonas exotoxin.

In an embodiment of the invention, said targeting carrier is an antigen, an antibody, a growth factor, a hormone, a lymphokine, a polypeptide cell recognition protein, a serum protein, a modified serum protein, an endorphin, an asialoglycoprotein, insulin, glucagon, luteinizing hormone, fibroblast growth factor, nerve growth factor, melanocyte stimulating hormone, bombesin, or a lectin.

In another embodiment, said targeting carrier is interfeukin-2, alpha transforming growth factor, or a peptide hormone.

According to another aspect of the present invention, an immunotoxin suitable for use with humans or animals comprising a toxin fused to an antibody capable of recognizing disease or disease-causing cells, wherein said toxin is a modified Pseudomonas exotoxin as defined above is provided.

According to a further aspect of the present invention, an expression plasmid comprising DNA encoding the targeting canrier-toxin conjugates is provided.

According to a further aspect, a host cell comprising said plasmid is provided.

According to another aspect, a host cell comprising DNA encoding the targeting carrier-toxin conjugates is provided.

According to another aspect, a process for producing said immunotoxin is provided comprising:
(i) transfecting a suitable host cell with a plasmid encoding the modified Pseudomonas exotoxin as defined above;
(ii) culturing said host cell under conditions so that the modified Pseudomonas exotoxin is produced, and
(iii) conjugating said modified exotoxin with an antibody to produce an immunotoxin.

In an embodiment of the invention, said modified Pseudomonas exotoxin comprises amino acid residues 385 to 613 of Pseudomonas exotoxin.

According to another aspect a process for producing said targeting carrier-toxin conjugate is provided, comprising transfecting a suitable host cell with a plasmid comprising a DNA sequence encoding said targeting carrier-toxin conjugate, and culturing said host cell under conditions so that a targeting carrier-Pseudomonas exotoxin conjugate is produced.

According to a further aspect, a composition suitable for administration to a mammal for achieving targeted cytotoxic activity in said mammal is provided, wherein the composition comprises said conjugate in a pharmaceutically acceptable carrier.

According to another aspect, the use of said conjugate for the preparation of a medicament for treating cancer is provided.

### Description of the Figures

Figure 1 shows the construction of the plasmids of the present invention used for the expression of different domains of PE.
Figure 2 is a simplified map of the different sized PE molecules produced as a part of the present invention.

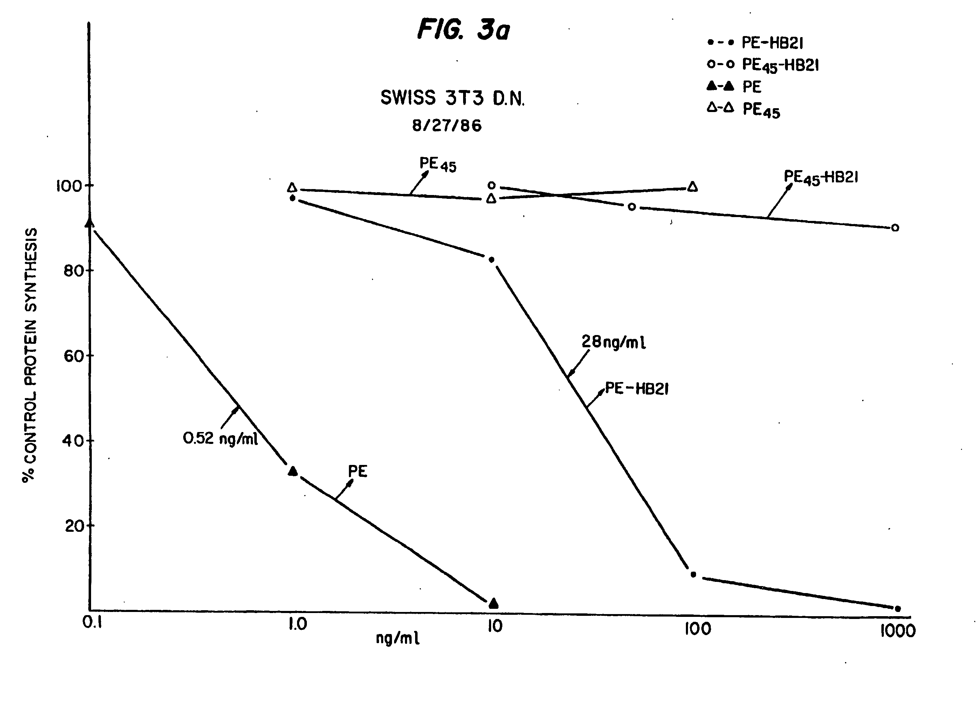

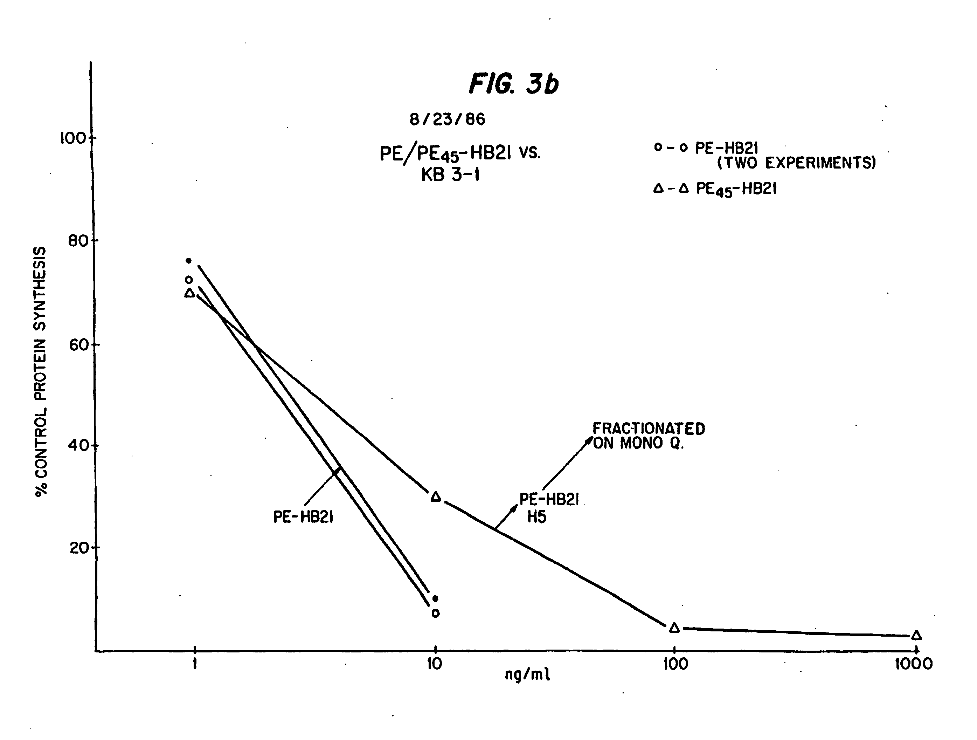

Figure 3 shows the effect of PE₄₅-HB21 and PE-HB21 on protein synthesis in human KB cells. PE₄₅ is the toxin protein lacking Domain I encoded by plasmid pJH8. Cells were incubated for 24 hours with the appropriate immunotoxin and then incubated with ³H-leucine for 1 hour. Incorporation of (³H)-leucine into protein was measured. In panel 3B Swiss 3T3 cells were incubated for 24 hours with either native pseudomonas toxin (PE), native pseudomonas toxin coupled to HB21, PE₄₅ alone, or PE₄₅ coupled to HB21. Cells were exposed to these agents for 24 hours and then protein synthesis measured for 1 hour as above.

### Specific Disclosure of the Invention

In the preferred embodiment; the present invention is the production of a modified form of Pseudomonas Exotoxin (PE) wherein the modification results in an active immunotoxin. The modified toxin and the immunotoxin made from it have markedly diminished non-specific toxicity on human and mouse cells in tissue culture and with greatly diminished toxicity in mice in vivo.

Genomic DNA is obtained by completely digesting a strain of Pseudomonas aeruginosa with EcoRI and PstI. Although any strain of P. aeruginosa is suitable for use in this invention, the preferred strain is PA103, a strain which produces a large amount of active toxin. DNA fragments ranging in size from 2.6 to 2.9 kb are isolated from the genomic DNA library, and ligated with a 2.7 kb DNA fragment from plasmid pUC13 cut with EcoRI and PstI. Plasmid pUC13 is commercially available from Boehringer Mannheim. A suitable host, preferably an E. coli strain, is then transformed with the ligated products (the preferred strain of E. coli is HB101, commercially available from Bethesda Research Laboratories). The 2.7 kb DNA fragment derived from ptoxETA, a plasmid which contains the PE structural gene, is used as a probe and plasmid DNA from several positive colonies were prepared and characterized by Southern blotting. The different sizes of recombinant PE are then expressed in a suitable host. A host which carries the bacteriophage T7 RNA polymerase gene in lysogenic and inducible form (BL21(DE₃)] is preferred, and is obtainable from Dr. F.W. Studier at Brookhaven National Laboratories. Also preferred are plasmids which carry the bacteriophage late T7 promoter and AMP^{r} and Shine-Dalgarno box--pAR2156, also obtainable from Dr. Studier.

As is shown in the Examples, the preferred plasmids are pJH8 and pJH17. pJH8 contains a DNA fragment of 5.1 kb and is capable of expressing Domains II, Ib, and III of PE. This plasmid is produced by partially cutting plasmid pJH4 with AvaI. The linearized DNA fragment is then completely cut with HindIII in order to obtain a 5.1 kb DNA fragment which has AvaI and HindIII sites at its ends. This fragment is then incubated with S1 nuclease to remove its cohesive ends, followed by ligation with T4 ligase to form plasmid pJH8.

Plasmid pJH17 contains a DNA fragment of 4.8 kb and is capable of expressing Domain III with the adjacent 20 amino acids of PE. This plasmid is produced by completely cutting plasmid pJH4 with HindIII and Apal. The 4.8 kb DNA fragment is then isolated and incubated with Klenow DNA polymerase I and dNTP to fill the cohesive ends, followed by ligation with T4 ligase.

### Expression of Recombinant Toxins in BL21 (DE₃).

BL21(DE₃) containing plasmids for expression of different sizes of PE is cultured in LB broth with 50 ug Ampicillin/ml at 37°C. When absorbance at 650 nm reaches 0.3, IPTG (isopropyl beta-D-thio-galactopyranoside) is added to the culture at a final concentration of 1mM. Cells are harvested 90 minutes later and analyzed for the amount of recombinant toxins by SDS-PAGE, immunoblotting, ADP-ribosylation, and cell killing experiments.

### SDS-PAGE and Immunoblotting

Cells pellets are dissolved in Laemmli buffer. Samples are boiled for 5 minutes prior to application to a 0.1% SDS, 10% acrylamide slab gel. For immunoblotting, samples after electrophoresis are transferred to a nitrocellulose paper, followed by reaction with antibody to PE, then a second antibody (goat anti-rabbit) and staining. The antibody to PE is obtained by hyperimmunizing rabbits with glutaraldehyde (0.2%) reacted PE (250 ug of PE per injection). An IgG fraction was prepared for immunoblotting.

### Assay of ADP-ribosylation Activity

Known procedures are used for assay of ADP-ribosylation activity. Briefly, rabbit reticulocyte preparations or wheat germ extracts enriched with elongation factor 2 (EF-2) are used as a source of EF-2. Assays (500 ul total volume) contain about 10 pmole of EP-2, 37 pmole of 14_{C-NAD} (0.06 u Ci), 0.25 to 1.25 ug of PE and buffer (40 mM DTT, 1 mM EDTA, and 50 mM Tris, pH 8.1). Activity is measured as pmoles of NAD transferred to EF-2 in 30 minutes. A standard curve of known concentrations of PE is established and used to determine the activity of PE in extracts from E. coli. After incubation for 30 minutes at 37°C, 0.5 ml 12% TCA is added to each assay mixture. The assay mixtures are then set in an ice bath for 15 minutes, followed by centrifugation at 4°C, 3,000 xg for 10 minutes. The pellet is washed with 1 ml 6% TCA and centrifuged as above. The pellet is then measured for ¹⁴C radioactivity in a liquid scintillation counter as the index of the ADP-ribosylation activity.

### Cell Cytotoxicity Test

Tests of the cytotoxic activity of the modified PE are performed in NIH 3T3 cell cultures and human KB cells. NIH 3T3 cells or KB cells are seeded 24 hours prior to the cytotoxicity test in a 24-well tissue culture plate at a density of 2 x 10⁴ cells per well. After incubation for 48 hours with various concentrations of PE or protein extracts isolated from BL21(DE₃) with plasmids which express different sizes of PE, the monolayers are stained with methylene blue to detect the surviving cells. The results are shown in Table 1.

### Inhibition of Protein Synthesis

Assays for the inhibition of protein synthesis by PE or PE with extracts from BL21(DE₃)/pJH8 are performed in Swiss 3T3 cell cultures. Swiss 3T3 cells are seeded one day before assay in 24-well tissue culture plates at a density of 10⁵ cells per well. Cells are washed once by replacing media with DMEM and 0.2% BSA before adding PE (100 ng/ml) or PE (100 ng/ml) with extracts which contained an excess amount of different sizes of PE (Table 2). After 15 minutes at 37 °C, the medium is then removed and replaced with fresh DMEM and 0.2% BSA. Four hours later, the rate of protein synthesis is assayed by adding [³H] leucine to the medium (to a final concentration of 2-4 uCi/ml) for 1 hour.

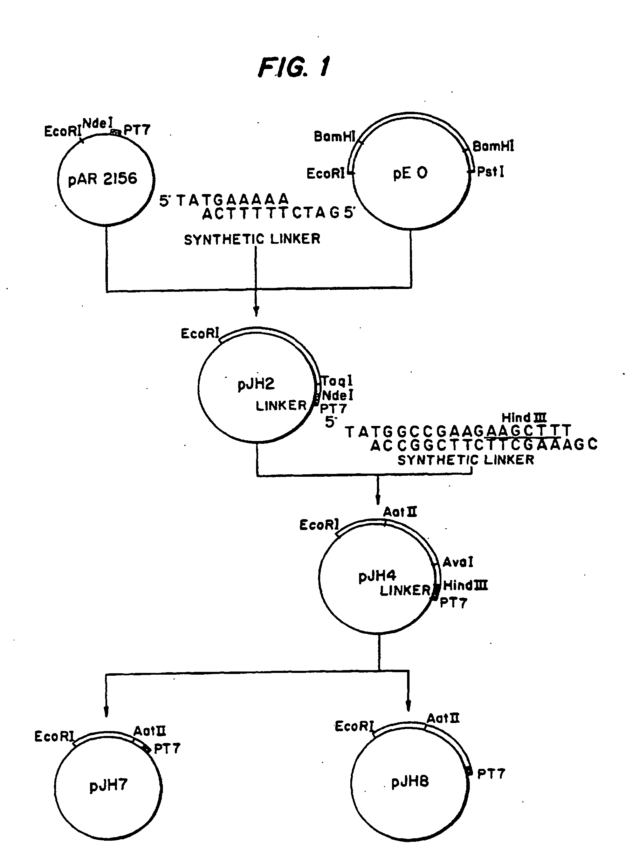

In the preferred embodiment of the invention, the structural domain of the Pseudomonas exotoxin gene is inserted into a T7 expression vector downstream of the ribosome binding site and its accompanying ATG initiation codon (as shown in Figure 1), as described above. To produce recombinant proteins, cells are grown at 37⁰C to an A₆₅₀ = 0.3. IPTG is then added at 1mM to induce T7 RNA polymerase and incubated for 2 hours. A series of plasmids are produced, as shown in Example 1.

Next, a clone is constructed which encodes a PE molecule without a leader sequence (pJH4) and in which a methionine is placed adjacent to the alanine at the amino terminus of the processed form of native PE (Table 1). The protein produced by pJH4 is designated Met-PE. Large amounts of Met-PE are produced upon induction by IPTG, representing 20% of total cell protein. ADP ribosylating activity equivalent to 0.1 mg native PE is found in the supernatant, and 0.2 mg in the pellet per mg of total cell protein. The PE molecule produced by pJH4 differs from native PE molecules by the presence of one extra methionine residue at the amino-terminus.

pJH8, produced from pJH4 as noted above, expresses a protein in which most or Domain Ia is deleted (retaining only the added methionine and three amino acids at the amino terminus. Immunoblotting shows that this protein is 45 kd, and is expressed at a concentration of approximately 0.04 mg/mg cell protein. High ADP ribosylating activity is exhibited when urea and DTT is excluded from the reaction mixture. In contrast to native PE (where the addition of urea and DTT activates ADP ribosylating activity), the addition of urea and DTT to pJH8 reduces (by about 30%) ADP ribosylating activity.

Because the ADP ribosylating activity of PE resides in the carboxyl end of the molecule, plasmid pJH17, constructed from pJH4 (as noted above), was produced. This plasmid contains Domain III and 20 amino acids from Domain Ib. Extracts from this plasmid contain high levels of ADP ribosylating activity--equivalent to 0.06 mg PE (Table 1). By immunoblotting, a 31 kd protein is detected, present at a concentration of 0.03 mg/mg cell protein.

None of the plasmids produced by the above-noted process (and described in the Examples), except for pJH1, pJH2, and pJH4, exhibit significant cell killing ability, even though many of these exhibit high enzymatic activity (Table 1). These plasmids are shown to prevent inhibition of protein synthesis or cell killing by native PE by exposing cells for 15 minutes to native PE at 0.1 ug/ml in the presence of 3-5 ug/ml of various modified toxins. The data in Table III shows that plasmids expressing either Domain Ia alone, or Domain I, half of Domain II and Domain III prevent PE from inhibiting protein synthesis.

### Animal Toxicity

Mice receiving native PE succumb due to liver destruction. The lethal dose for a 20 gram mouse is 0.1-0.2 ug. The data in Table IV shows that PE with a deletion of Domain Ia exhibits greatly diminished toxicity in mice. Mice were injected I.P. with either native PE or lacking Domain Ia. All mice receiving 1.0 ug of native PE and one-halt receiving 0.2 ug PE were dead at 48 hours. Two of three mice receiving 50 ug of PE Ia died; all receiving 20 ug of PE Ia lived; and all mice receiving 5 ug PE Ia lived. Thus, PE Ia is more than 100 times less toxic on a weight basis than native PE.

### Gene Fusions Using Recombinant Modified Pseudomonas Exotoxin.

The gene for modified PE of this invention specifically contained in pJH8 was fused with DNA sequences encoding the human alpha transforming growth factor (a-TGF) or human interleukin 2 (IL-2). See Examples 7 and 8 for the specifics of these gene fusions. Modified PE is suitable for use in fusions with any peptide hormone, growth factor, or other polypeptide cell recognition protein for which a specific receptor exists on cells. A few examples include: insulin, glucagon, endorphins, growth hormone, melanocyte stimulating hormone, transferrin, bombesin, low density lipoprotein, luteinizing hormone, and asialoglycoproteins.

### Examples

Example 1. As shown in Table 1, the process of the present invention was used to construct several plasmids containing fusions of the different sizes of PE structural gene and the T7 late promoter. pJH2 contains the intact PE structural gene with a segment coding for a modified leader sequence in front. pJH4 contains the intact PE structural gene with the addition of a methionine codon at the amino-terminus. pJH7 contains the gene encoding structural Domain III of PE (amino acid 405-613). pJH8 contains the gene encoding structural Domain II, Ib, and III-of PE (amino acid 253-613. pJH13 encodes PE with a deletion of the first half of structural domain II encompassing amino acids 253-307. pJH14 encodes structural domain Ia of PE (amino acids 1-252). pJH17 encodes structural domain III with an additional adjacent sequence of 20 amino acids from Domain Ib (amino acids 385-613).

pJH1 was constructed by partially cutting the native PE (pE 0) with BamHI, and the linear form of DNA was eluted and completely cut with EcoRI. The 2.0kb DNA fragment derived from pE 0, which has BamHI and EcoRI at the two ends, was then inserted into pAR 2156, which had been completely cut with BamHI and EcoRI.

pJH2 was constructed by partially cutting pJH1 with BamHI. The linear form of DNA was isolated and completely cut with NdeI. The 610 kb DNA fragment was saved to construct pJH2 by ligating it with synthetic oligonucleotide duplex

pJH4 was constructed by partially cutting pJH2 with TaqI. The linearized DNA (610 kb) was isolated and completely cut with NdeI. The largest DNA fragment (5.9 kb) was separated and ligated with synthetic oligonucleotide duplex (which contains a HindIII site--AAGCTT). TTCGAA

pJH7 was constructed by partially cutting pJH4 with AatII. The linearized DNA fragment (5.9 kb) was then completely cut with HindIII. The 4.7 kb DNA fragment which has AatII and HindIII sites at its ends after separation was incubated with S1 nuclease to remove the cohesive ends, followed by ligation with T4 ligase.

pJH8 was constructed as described in the Specific Disclosure.

pJH13 was constructed by partially cutting pJH4 with AvaI. The linearized DNA fragment was then completely cut with EcoRI. The 4.7 kb DNA which has AvaI and EcoRI cut at both ends was incubated with S1 to remove cohesive ends (DNA fragment 1). pJH4 was partially cut with SalI. The linearized DNA fragment was then completely cut with EcoRI. The 1.0 kb DNA fragment which has SalI and EcoRI sites at the ends was incubated with Klenow DNA polymerase I and dNTP to fill the cohesive ends (DNA fragment 2). DNA fragment 1 (4.7 kb) and DNA fragment 2 (1.0 kb) were ligated at 4° overnight.

pJH14 was constructed by partially cutting pJH4 with AvaI. The linearized DNA fragment was then completely cut with EcoRI. The 4.7 kb DNA which has AvaI and EcoRI sites at its ends was incubated with S1 to remove the cohesive end, followed by ligation with T4 ligase.

pJH17 was constructed as described in the Specific Disclosure.

Example 2. The amount and activity of the recombinant toxins (produced by the plasmids described in Example 1) were measured by SDS-PAGE, ADP-ribosylation, and cell killing ability. The results are tabulated in Table 1.

Example 3. Experiments were conducted to examine the characteristics of the recombinant toxicity of the present invention. The results are shown in Table 2.

Example 4. The effect of various deletions on cell protein synthesis was determined, in the presence and absence of native PE. The results are shown in Table 3. Structural Domain Ia (pJH14) and structural domain I, half of II, and III (pJH13) were extracted from the pellet of the sonicated cells with 8M urea. 10 ul of each extract equivalent to 3-5 ug of recombinant proteins was used in each assay. Structural II, Ib, and III (pJH8) were present in the supernatant of the sonicated BL21(DE₃)/pJH8 cells. 10 ul of extract equivalent to 2 ug of recombinant toxin was used. Cells were treated as indicated in Table 3 for 15 minutes with and without native PE at 0.1 ug/ml followed by 1 ml DMEM washing; incubated for 4 hours in DMEM and 0.2% BSA, then incubated with ³H-leucine for 1 hour.

Example 5. As shown in Table 4, the dose causing death in mice was determined by injecting Balb/c mice I.P. with various amounts of PE or PE Ia contained in 1.0 ml of sterile saline and 10 mgs/ml sterile human albumin. The animals were monitored daily for two weeks. All deaths occured at 48 hours.

Example 6. As shown in Figure 3, an immunotoxin composed of the 45 kD protein produced by pJH8 conjugated by a disulfide bond to an antibody to the human transferrin receptor (PE₄₅-HB21) kills human cells expressing the transferrin receptor (ID₅₀ 3 ng/ml). It has little or no effect on mouse cells which do not express the human transferrin receptor at 1000 ng/ml, whereas native PE conjugated by a sulfide bond to HB21 nonspecifically kills mouse cells at an ID₅₀ of 29 ng/ml. The data in Figure 3 indicate the non-specific toxicity of PE₄₅-HB21 is 100-fold less than PE-H321.

The molecular weight of PE₄₅ was subsequently re-determined. The molecular weight was then found to be 40,000.

Example 7. Construction of alpha Transforming Growth Factor-PE fusion gene. pJH8 was treated with Tth 111I and SphI to construct a smaller plasmid pVC8 which has fewer AvaII sites. pVC8 was partially cut with AvaII and ligated to a synthetic oligonucleotide (30bp) which contains a STuI site, a Tth lllI site, and a stop codon in order to create pVC31. pVC31 was cut with Tth 111I, and filled in with a Klenow fragment, a fragment of DNA polymerase, and ligated to a blunt ended clone containing the alpha-TGF gene (pVC 33). The alpha-TGF DNA, p-hTGF-10-925 (Derynck etal, Cell, 38:287-297 (19884)) was cut with EcoRI and BglI to give a 322 bp fragment which was isolated and cut with Fnu 4HI and treated with T4 polymerase to give a 152 bp fragment which in turn was ligated to pVC31 to create PE-alphaTGP fusion gene. When expressed in E. coli B121, this plasmid produces a protein that reacts with antibodies to alpha-TGF and to PE and has a molecular weight of 51,000.

### Example 8. Construction of IL-2-PE Fusion Gene.

pVC8 was cut with AvaII at position 1190, the 3.6 fragment isolated, its single stranded ends filled with Klenow enzyme, and dephosphorylated to produce fragment I. Clone PST-5 [Gallo et al, PNAS, 81:2543-2547 (1984)] was cut with PstI to produce a 1 kD fragment of IL-2 which was then cut with Bsp 1286 at positions 105 and 669 and treated with T4 polymerase to fill up the ends. The 564 bp fragment was ligated to fragment I to create pHL-1 and transformed into BL21 expression cells. Upon induction, a 60 kD protein was produced that reacts with antibodies to IL-2 and to PE, and contains ADP-ribosylating activity.

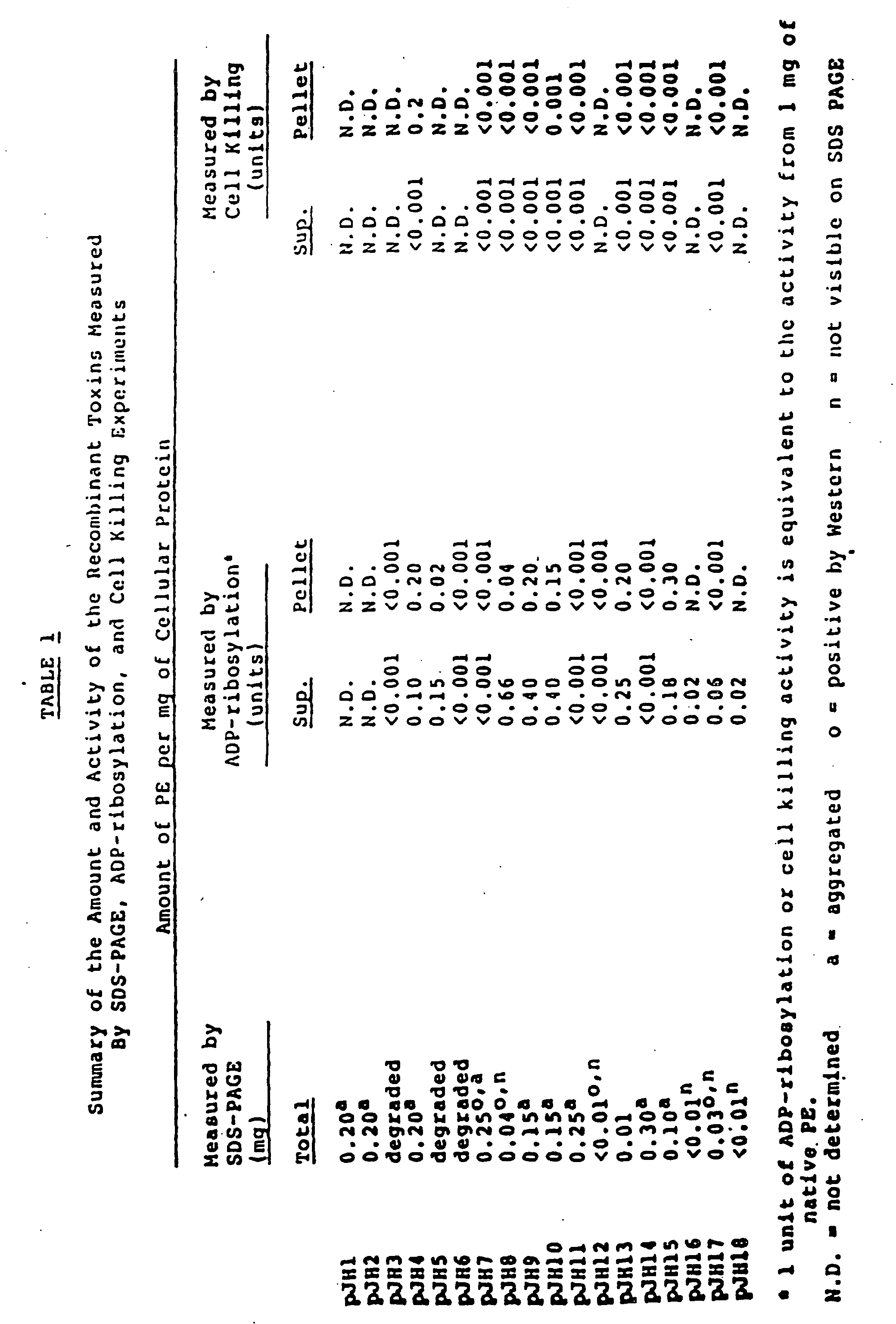

**TABLE 2**

| **Construction** | **Domain Present** | **Protein Size** |
|---|---|---|
| pJH4 | met, I, II, III | 68 kd |
| pJH7 | III | 28 kd |
| pJH8 | II, Ib, III | 45 kd |
| pJH13 | I, half of II, III | 63 kd |
| PJH14 | Ia | 32 kd |
| pJH17 | 20 a.a. of Ib, III | 31 kd |

**TABLE 3**

| | ^{**3**}**H-leucine in corporation (cpm x 10**^{**3**}**)** | |
|---|---|---|
| **Domain Tested** | **without PE** | **with PE (I, II, III)** |
| none | 11.2 ± .1 | 2.3 ± .2 |
| Ia | 11.5 ± .15 | 9.4 ± .3 |
| II, Ib, & III | 11.7 ± .15 | 2.4 ± .2 |
| I, 1/2 II, III | 10.7 ± .15 | 9.2 ± .2 |
| 8 M urea (10 ul) | 11.1 ± .1 | 2.9 ± .2 |

**TABLE 4**

| **Toxin** | **Dose (ug)** | **Deaths** |
|---|---|---|
| PE₄₅ | 50 | 2/3 |
| PE₄₅ | 20 | 0/3 |
| PE₄₅ | 5 | 0/3 |
| PE | 10 | 3/3 |
| PE | 1.0 | 3/3 |
| PE | 0.3 | 3/3 |
| PE | 0.2 | 1/3 |
| PE | 0.1 | 0/3 |

### Statement of Deposit

The following plasmids have been deposited in the American Type Culture Collection in Rockville, Maryland, under the respective ATCC numbers, prior to the filing of this application, and at issuance of this application into a patent will be maintained for a term of thirty (30) years from the date of deposit or five (5) years after the last request for such deposit or for the effective life of the patent, whichever is longest. The deposits will be replaced if the cultures mutate or become nonviable during the term of the deposit:

| | |
|---|---|
| pJH12 | ATCC 67205 |
| pHL-1 | ATCC 67206 |
| pVC33 | ATCC 67207 |
| pJH8 | ATCC 67208 |
| pJH14 | ATCC 67209 |

## Claims

1. A targeting carrier-toxin conjugate suitable for use with humans or mammals comprising a toxin fused to a targeting carrier wherein said toxin is a modified Pseudomonas exotoxin selected from:
(i) an exotoxin comprising the amino acid residues 1 to 30 and 308 to 613 of Pseudomonas exotoxin;
(ii) an exotoxin comprising the amino acid residues 1 to 252 and 308 to 613 of Pseudomonas exotoxin; and
(iii) an exotoxin comprising the amino acid residues 385 to 613 of Pseudomonas exotoxin.

2. The conjugate of claim 1, wherein said targeting carrier is an antigen, an antibody, a growth factor, a hormone, a lymphokine, a polypeptide cell recognition protein, a serum protein, a modified serum protein, an endorphin, an asialoglycoprotein, insulin, glucagon, luteinizing hormone, fibroblast growth factor, nerve growth factor, melanocyte stimulating hormone, bombesin, or a lectin.

3. The conjugate of claim 1, wherein said targeting carrier is interleukin-2, alpha transforming growth factor, or a peptide hormone.

4. An immunotoxin suitable for use with humans or animals comprising a toxin fused to an antibody capable of recognizing disease or disease-causing cells, wherein said toxin is a modified Pseudomonas exotoxin as defined in claim 1.

5. An expression plasmid comprising DNA encoding any one of the targeting carrier-toxin conjugates of claims 1 to 4.

6. A host cell comprising DNA encoding any one of the targeting carrier-toxin conjugates of claims 1 to 4.

7. A host cell comprising a plasmid of claim 5.

8. A process for producing an immunotoxin according to claim 4, comprising:
(i) transfecting a suitable host cell with a plasmid encoding the modified Pseudomonas exotoxin as defined in claim 1,
(ii) culturing said host cell under conditions so that the modified Pseudomonas exotoxin is produced, and
(iii) conjugating said modified exotoxin with an antibody to produce an immunotoxin.

9. The process of claim 8, wherein said modified Pseudomonas exotoxin comprises amino acid residues 385 to 613 of Pseudomonas exotoxin.

10. A process for producing a targeting camer-toxin conjugate according to claim 1, comprising transfecting a suitable host cell with a plasmid comprising a DNA sequence encoding said targeting carrier-toxin conjugate and culturing said host cell under conditions so that a targeting carrier-Pseudomonas exotoxin conjugate is produced.

11. A composition suitable for administration to a mammal for achieving targeted cytotoxic activity in said mammal, wherein the composition comprises a conjugate according to any one of claims 1 to 4 in a pharmaceutically acceptable carrier.

12. Use of a conjugate according to any one of claims 1 to 4 for the preparation of a medicament for treating cancer.

## Patentansprüche

1. Konjugat aus einem zielorientierten Träger und Toxin, welches für die Verwendung bei Menschen oder Säugetieren geeignet ist, umfassend ein Toxin, welches an einen zielorientierten Träger fusioniert ist, worin das Toxin ein modifiziertes Pseudomonas-Exotoxin ist, ausgewählt aus:
(i) einem Exotoxin, umfassend die Aminosäurereste 1 bis 30 und 308 bis 613 des Pseudomonas-Exotoxins;
(ii) einem Exotoxin, umfassend die Aminosäurereste 1 bis 252 und 308 bis 613 des Pseudomonas-Exotoxins; und
(iii) einem Exotoxin, umfassend die Aminosäurereste 385 bis 613 des Pseudomonas-Exotoxins.

2. Konjugat nach Anspruch 1, worin der zielorientierte Träger ein Antigen, ein Antikörper, ein Wachstumsfaktor, ein Hormon, ein Lymphokin, ein Polypeptid-Zellerkennungsprotein, ein Serumprotein, ein modifiziertes Serumprotein, ein Endorphin, ein Asialoglykoprotein, Insulin, Glucagon, luteinisierendes Hormon, Fibroblastenwachstumsfaktor, Nervenwachstumsfaktor, Melanocyten-stimulierendes Hormon, Bombesin oder ein Lectin ist.

3. Konjugat nach Anspruch 1, worin der zielorientierte Träger Interleukin-2, α-transformierender Wachstumsfaktor oder ein Peptidhormon ist.

4. Immuntoxin, welches für die Verwendung bei Menschen oder Tieren geeignet ist, umfassend ein Toxin, welches an einen Antikörper fusioniert ist, der fähig ist, erkrankte oder Krankheit verursachende Zellen zu erkennen, worin das Toxin ein modifiziertes Pseudomonas-Exotoxin, wie in Anspruch 1 definiert, ist.

5. Expressionsplasmid, umfassend DNA, welche eines der Konjugate aus einem zielorientierten Träger und Toxin nach den Ansprüchen 1 bis 4 kodiert.

6. Wirtszelle, umfassend DNA, welche eines der Konjugate aus einem zielorientierten Träger und Toxin nach den Ansprüchen 1 bis 4 kodiert.

7. Wirtszelle, umfassend ein Plasmid nach Anspruch 5.

8. Verfahren zum Herstellen eines Immuntoxins nach Anspruch 4, umfassend:
(i) das Transfizieren einer geeigneten Wirtszelle mit einem Plasmid, welches das modifizierte Pseudomonas-Exotoxin, wie in Anspruch 1 definiert, kodiert,
(ii) das Kultivieren der Wirtszelle unter Bedingungen, so dass das modifizierte Pseudomonas-Exotoxin produziert wird, und
(iii) das Konjugieren des modifizierten Exotoxins mit einem Antikörper, um ein Immuntoxin herzustellen.

9. Verfahren nach Anspruch 8, worin das modifizierte Pseudomonas-Exotoxin die Aminosäurereste 385 bis 613 des Pseudomonas-Exotoxins umfasst.

10. Verfahren zum Herstellen eines Konjugats aus einem zielorientierten Träger und Toxin nach Anspruch 1, umfassend das Transfizieren einer geeigneten Wirtszelle mit einem Plasmid, umfassend eine DNA-Sequenz, welche das Konjugat aus einem zielorientiertem Träger und Toxin kodiert, und das Kultivieren der Wirtszelle unter Bedingungen, so dass ein Konjugat aus einem zielorientierten Träger und Pseudomonas-Exotoxin produziert wird.

11. Zusammensetzung, welche für die Verabreichung an ein Säugetier zum Erreichen einer zielorientierten zytotoxischen Aktivität in dem Säugetier geeignet ist, worin die Zusammensetzung ein Konjugat nach einem der Ansprüche 1 bis 4 in einem pharmazeutisch annehmbaren Träger umfasst.

12. Verwendung eines Konjugats nach einem der Ansprüche 1 bis 4 für die Herstellung eines Medikaments zum Behandeln von Krebs.

## Revendications

1. Conjugué support de ciblage-toxine approprié à une utilisation chez les humains ou les mammifères, comprenant une toxine fusionnée à un support de ciblage, dans laquelle ladite toxine est une exotoxine de Pseudomonas modifiée choisie parmi :
(i) une exotoxine comprenant les résidus d'acides aminés 1 à 30 et 308 à 613 d'exotoxine de Pseudomonas ;
(ii) une exotoxine comprenant les résidus d'acides aminés 1 à 252 et 308 à 613 d'exotoxine de Pseudomonas ; et
(iii) une exotoxine comprenant les résidus d'acides aminés 385 à 613 d'exotoxine de Pseudomonas.

2. Conjugué de la revendication 1, dans lequel ledit support de ciblage est un antigène, un anticorps, un facteur de croissance, une hormone, une lymphokine, un polypeptide, une protéine de reconnaissance cellulaire, une protéine sérique, une protéique sérique modifiée, une endorphine, une asialoglycoprotéine, l'insuline, le glucagon, l'hormone lutéïnisante, le facteur de croissance des fibroblastes, le facteur de croissance des nerfs, l'hormone stimulant les mélanocytes, la bombésine ou une lectine.

3. Conjugué de la revendication 1, dans lequel ledit support de ciblage est l'interleukine 2, le facteur de croissance transformant alpha, ou une hormone peptidique.

4. Immunotoxine appropriée à une utilisation chez les humains ou les animaux, comprenant une toxine fusionnée à un anticorps capable de reconnaître une maladie ou une cellule provoquant une maladie, dans laquelle ladite toxine est une exotoxine de Pseudomonas modifiée comme défini à la revendication 1.

5. Plasmide d'expression comprenant un ADN codant l'un quelconque des conjugués support de ciblage-toxine des revendications 1 à 4.

6. Cellule hôte comprenant un ADN codant l'un quelconque des conjugués support de ciblage-toxine des revendications 1 à 4.

7. Cellule hôte comprenant un plasmide de la revendication 5.

8. Procédé de production d'une immunotoxine selon la revendication 4, comprenant :
(i) la transfection d'une cellule hôte appropriée par un plasmide codant l'exotoxine de Pseudomonas modifiée telle que définie dans la revendication 1,
(ii) la culture de ladite cellule hôte dans des conditions telles que l'exotoxine de Pseudomonas modifiée soit produite, et
(iii) la conjugaison de ladite exotoxine modifiée avec un anticorps pour produire une immunotoxine.

9. Procédé de la revendication 8, dans lequel ladite exotoxine de Pseudomonas modifiée comprend les résidus d'acides aminés 385 à 613 d'exotoxine de Pseudomonas.

10. Procédé de production d'un conjugué support de ciblage-toxine selon la revendication 1, comprenant la transfection d'une cellule hôte appropriée par un plasmide comprenant une séquence d'ADN codant ledit conjugué support de ciblage-toxine et la culture dé ladite cellule hôte dans des conditions telles qu'un conjugué support de ciblage-exotoxine de Pseudomonas soit produit.

11. Composition appropriée à l'administration à un mammifère pour obtenir une activité cytotoxique ciblée chez ledit mammifère, laquelle composition comprenant un conjugué selon l'une quelconque des revendications 1 à 4 dans un support pharmaceutiquement acceptable.

12. Utilisation d'un conjugué selon l'une quelconque des revendications 1 à 4 pour la préparation d'un médicament pour le traitement du cancer.
